# EUROPÄISCHE PATENTSCHRIFT

(11) **EP 0 855 395 B1**
(45) Veröffentlichungstag und Bekanntmachung des Hinweises auf die Patenterteilung: **17.04.2002**
(21) Anmeldenummer: 98250021.7
(22) Anmeldetag: 22.01.1998
(51) Int. Cl.: C07D 305/12, C07C 69/708, C07C 47/198, C07C 47/277

(54) **Verfahren für die Herstellung von Oxetan-2-onen und Zwischenprodukte**
Process for the preparation of oxetan-2-ones and intermediates
Procédé pour la préparation d'oxétan-2-ones et produits intermédiaires

(30) Priorität: 23.01.1997 DE 19703713
(43) Veröffentlichungstag der Anmeldung: 29.07.1998
(73) Patentinhaber: Asca GmbH, Angewandte Synthesechemie Adlershof, 12489 Berlin (DE)
(72) Erfinder: Schick, Hans, Prof. Dr., 10115 Berlin (DE); Wedler, Christine, Dr., 10115 Berlin (DE)
(74) Vertreter: Walter, Wolf-Jürgen

(56) Entgegenhaltungen:
- WO-A-93/23379
- WO-A-94/04491
- WO-A-94/10133
- P. BARBIER ET AL.: HELVETICA CHIMICA ACTA, Bd. 70, 1987, Seiten 1412-8, XP002064047
- S. HANESSIAN ET AL.: JOURNAL OF ORGANIC CHEMISTRY, Bd. 58, 1993, Seiten 7768-81, XP002064048
- N. K. CHADHA ET AL.: JOURNAL OF ORGANIC CHEMISTRY, Bd. 56, 1991, Seiten 4714-8, XP002064049
- I. FLEMING ET AL.: TETRAHEDRON LETTERS, Bd. 31, Nr. 25, 1990, Seiten 3645-8, XP002064050
- A. POMMIER ET AL.: SYNTHESIS, Dezember 1994, Seiten 1294-1300, XP002064051

## Beschreibung

Die Erfindung betrifft ein neues dreistufiges Verfahren für die Herstellung von Oxetan-2-onen sowie Zwischenprodukte dafür.

3,4-Disubstituierte Oxetan-2-one dienen als zentrale Zwischenprodukte für die Herstellung von pharmakologisch wirksamen β-Lactonen, von denen die Enzyminhibitoren Lipstatin, Tetrahydrolipstatin, Esterastin, Tetrahydroesterastin und Valilacton sowie strukturell verwandte Verbindungen von besonderem Interesse sind.

Für ihre Herstellung in enantiomeren- und diastereomerenreiner Form werden verschiedene Verfahren benutzt, die hinsichtlich ihrer Stufenzahl, hinsichtlich des Materialeinsatzes, hinsichtlich des Reinigungsaufwandes und/oder hinsichtlich der Gesamtausbeute nicht befriedigen. Als Beispiele seien genannt:

Die Synthese von Barbier, Schneider und Widmer (*Helv*. *Chim*. *Acta* **1987**, *70,* 1412-1418) für (3*S*,4*S*)-3-Hexyl-4-[(2*R*)-2-hydroxytridecyl]oxetan-2-on liefert ausgehend von (*R*)-3-Hydroxytetradecansäure-methylester nach 6 Stufen eine Gesamtausbeute von 6 %.

Die Synthese von Pommier, Pons, Kocienski und Wong (*Synthesis* **1994,** 1294-1300) für dasselbe β-Lacton liefert ausgehend von (*R*)-3-Hydroxytetradecansäure-methylester in 5 Stufen eine Gesamtausbeute von 40 %. Dazuzurechnen sind weitere drei Stufen mit einer Gesamtausbeute von 25 % am Anfang der Synthese für die aufwendige Herstellung von 3-Hexyl-3-trimethylsilylketen.

Die Synthese von Uskokovic et al. (*J*. *Org. Chem.* **1991,** *56*, 4714-4718) für (3*S*,4*S*)-3-Hexyl-4-[(2*S*)-2-hydroxytridecyl]oxetan-2-on liefert ausgehend von Cyclopentadien in 17 Stufen eine Gesamtausbeute von nur 1 %.

Die Synthese von Hanessian, Tehim und Chen (*J*. *Org. Chem.* **1993,** *58,* 7768-7781) für (3*S*,4*S*)-3-Hexyl-4-[(2*R*)-2-hydraxytridecyl]oxetan-2-on liefert ausgehend von Dodecanal in 9 Stufen eine Gesamtausbeute von 35 %.

Die Synthese von Fleming und Lawrence (*Tetrahedron Lett.* **1990,** *31*, 3645-3648) für (3*S*,4*S*)-3-Hexyl-4-[(2*S*)-2-hydroxytridecyl]oxetan-2-on liefert ausgehend von 3-(Dimethylphenylsilyl)propinsäure in 19 Stufen eine Gesamtausbeute von 10 %.

Der Erfindung liegt die Aufgabe zugrunde, ein Verfahren zur wirtschaftlichen Herstellung von Oxetan-2-onen über neue Zwischenprodukte zu entwickeln, wobei die Oxetan-2-one als Zwischenprodukte für Enzyminhibitoren und strukturell verwandte Verbindungen dienen. Dieses Verfahren soll möglichst wenige Reaktionsstufen umfassen, billige, einfach einzuführende und abzuspaltende Schutzgruppen verwenden und eine Trennung von Diastereomeren durch einfache Trennmethoden ermöglichen.

Diese Aufgabe wird erfindungsgemäß durch Herstellung von Oxetan-2-onen der allgemeinen Formel **I** 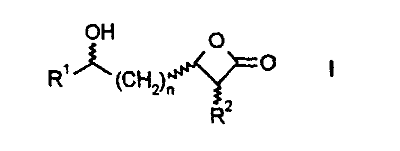 gelöst, wobei Ester der allgemeinen Formel **II** 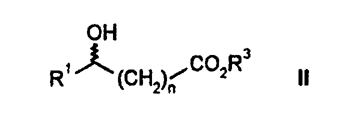 in der R¹ einen Alkyl-, Alkenyl-, Alkadienyl- oder Alkinylrest mit bis zu 20 Kohlenstoffatomen bedeutet, in welchem die Kohlenstoffkette durch 1 bis 3 Halogenatome, eine Alkylgruppe, eine Arylgruppe oder eine Arylalkylgruppe substituiert oder durch ein Sauerstoffatom, ein Schwefelatom oder eine 1,4-Arylengruppe unterbrochen sein kann, R³ Alkyl bedeutet und n den Wert 0 oder 1 hat, mit Enolethern der allgemeinen Formel **III** 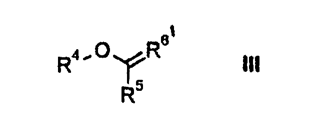 in der R⁴ Alkyl, R⁵ Wasserstoff oder Alkyl , R^{6'} Alkyliden, vorzugsweise Methylen oder Ethyliden oder R⁵ und R^{6'} zusammen Alkyliden, vorzugsweise CH(CH₂)_{m'} mit m'= 3 bis 14 bedeuten, in einer säurekatalysierten Reaktion zu Verbindungen der allgemeinen Formel **IV** 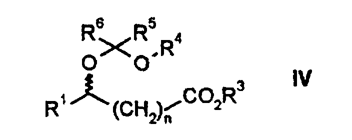 umgesetzt werden, in der R¹, R³, R⁴, R⁵ und n die oben genannte Bedeutung haben, R⁶ Alkyl, vorzugsweise Methyl oder Ethyl, oder R⁵ und R⁶ zusammen (CH₂)ₘ mit m = 4 bis 15 bedeuten.

Die für R¹ genannten Reste haben vorzugsweise 4 bis 18 Kohlenstoffatome,

Die für R¹ genannten Substituenten sind vorzugsweise Fluor-, Chlor- oder Bromatome, C₁-C₆-Alkyl, Phenyl, Methoxyphenyl, Halogenphenyl, Naphthyl, Benzyl, oder Ethylphenyl.

R³ und R⁶ sind vorzugsweise C₁-C₅-Alkyl, insbesondere Methyl oder Ethyl.

R⁴ ist vorzugsweise C₁-C₅-Alkyl, insbesondere Methyl.

Wenn R⁵ Alkyl bedeutet, ist es vorzugsweise C₁-C₅-Alkyl, insbesondere Methyl oder Ethyl.

Die 1,4-Arylengruppe, mit der die Kette von R¹ unterbrochen sein kann, ist vorzugsweise eine 1,4-Phenylengruppe.

Die Verbindungen der allgemeinen Formel **IV** werden durch Reduktion in einem organischen Lösungsmittel wie Toluol oder Dichlormethan, vorzugsweise mit Diisobutylaluminiumhydrid, in einen Aldehyd der Formel **V** 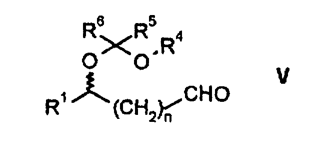 überführt, in der R¹, R⁴, R⁵, R⁶ und n die oben genannte Bedeutung haben.

Die Verbindungen der allgemeinen Formel **V** werden mit einem Metallenolat der allgemeinen Formel **VI** 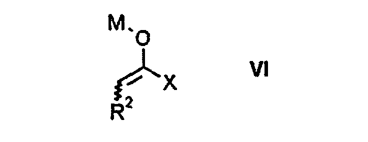 in der R² eine Arylamino-, Alkoxy-, Aryloxy-, Alkylsulfanyl-, Arylsulfanyl- oder Halogengruppe oder einen Alkyl-, Alkenyl-, Alkadienyl- oder Alkinylrest mit bis zu 20 Kohlenstoffatomen bedeutet, in welchem die Kohlenstoffkette durch 1 bis 3 Halogenatome, eine Alkylgruppe, eine Arylgruppe oder eine Arylalkylgruppe substituiert oder durch ein Sauerstoffatom, ein Schwefelatom oder eine 1,4-Arylengruppe unterbrochen sein kann, X Fluor oder eine gegebenenfalls substituierte Aryloxy-, Arylsulfanyl- oder stickstoffhaltige Heteroarylgruppe, vorzugsweise eine 1-Benzotriazolyl- oder Phenoxygruppe ist, und M für das Äquivalent eines ein-, zwei-, drei- oder vierwertiges Metalls wie Li, MgBr, ZnCl oder Ti(OPr')₃ steht, zu Verbindungen der allgemeinen Formel **I** 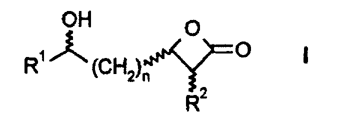 umgesetzt, in der R¹, R² und n die oben genannte Bedeutung haben.

Die Herstellung der Oxetan-2-one ist in dem beiliegenden Reaktionsschema nochmals erläutert.

Durch übliche chromatographische Trennmethoden, z.B. Säulenchromatographie mit oder ohne Druck an unterschiedlichen Adsorbentien und/oder Umkristallisation lassen sich die erhaltenen Gemische von Diastereomeren der allgemeinen Formel **I** trennen.

Bevorzugt werden :
A. Verfahren zur Herstellung von Verbindungen der allgemeinen Formel **I** dadurch gekennzeichnet, daß diese Verbindungen als Gemisch von Diastereomeren oder in diastereomerenreiner, enantiomerenreiner oder racemischer Form erhalten werden.
B. Verfahren dadurch gekennzeichnet, daß eine Verbindung der allgemeinen Formel **II** mit 2-Methoxypropen in Gegenwart von Pyridinium-*p*-toluolsulfonat mit oder ohne organisches Lösungsmittel bei einer Temperatur zwischen -20 und 35 °C, vorzugsweise bei 20 bis 25 °C umgesetzt wird.
C. Verfahren dadurch gekennzeichnet, daß die Reduktion eines Esters der allgemeinen Formel **IV** zu einem Aldehyd der allgemeinen Formel **V** mit Diisobutylaluminiumhydrid bei einer Temperatur von -20 bis -90 °C, vorzugsweise bei -70 °C durchgeführt wird.
D. Verfahren dadurch gekennzeichnet, daß die Umsetzung eines Aldehyds der allgemeinen Formel **V** mit einem Metallenolat der allgemeinen Formel **VI** zu einem Oxetan-2-on der allgemeinen Formel **I** bei einer Temperatur von -20 bis -100 °C, vorzugsweise bei - 90 °C erfolgt.

Das erfindungsgemäße Verfahren zur Herstellung von Oxetan-2-onen zeichnet sich durch eine vergleichsweise hohe Ausbeute bei geringer Stufenzahl sowie den Einsatz vergleichsweise billiger Reagentien aus. Die erfindungsgemäßen Acetalschutzgruppen lassen sich in einer quantitativ verlaufenden säurekatalysierten Reaktion einführen und sind so labil, daß sie bei der sauren Aufarbeitung der Oxetan-2-one ohne zusätzlichen Aufwand wieder abgespalten werden. Als weiterer Vorteil des erfindungsgemäßen Verfahrens ist anzusehen, daß aus einem (*S*)-3-Hydroxycarbonsäureester das Diastereomer als Hauptprodukt gebildet wird, das für die Veresterung zu Lipstatin-analogen Verbindungen keine Inversion der Hydroxylgruppe in der Seitenkette erfordert. Dadurch kann für diese Veresterung die vergleichsweise material- und kostenaufwendige Mitsunobu-Reaktion umgangen werden.

Gegenstand der Erfindung sind weiterhin die als Zwischenprodukte in dem erfindungsgemäßen Verfahren erhältlichen Verbindungen der allgemeinen Formeln **IV** und **V** wie in Ansprüche 6 und 8 definiert, als Gemische von Diastereomeren oder in diastereomerenreiner, enantiomerenreiner oder racemischer Form.

### Beispiel 1

### (±)-3-(1-Methyl-1-methoxyethoxy)tetradecansäure-methylester (rac-VII)

5.16 g (20 mmol) (±)-3-Hydroxytetradecansäure-methylester werden in 20 ml 2-Methoxypropen gelöst und mit 50 mg Pyridinium-*p*-toluolsulfonat versetzt. Nach 45-minütigem Stehen bei Raumtemperatur wird das Reaktionsgemisch mit 20 ml Diethylether versetzt und einmal mit 4 ml gesättigter wäßriger Natriumhydrogencarbonat-Lösung und zweimal mit je 4 ml Wasser gewaschen. Die organische Phase wird über Natriumsulfat getrocknet und nach Abfiltrieren des Trockenmittels unter vermindertem Druck vom Lösungsmittel und überschüssigem 2-Methoxypropen befreit. Der als Rückstand verbleibende (±)-3-(1-Methyl-1-methoxyethoxy)tetradecansäure-methylester (*rac*-**VII**) ist für die Weiterverarbeitung genügend rein [¹³C-NMR (500 MHz) einer analysenreinen Probe: δ = 13.85, 22.02, 22.05, 24.36, 24.86, 28.67, 28.91, 28.97, 28.98, 29.03, 30.93, 31.27, 35.13, 40.21, 48.46, 51.15, 67.67, 100.35, 171.62. - C₁₉H₃₈O₄ (330.5): berechnet C 69.05, H 11.59, gefunden C 69.20, H 19.38].

### Beispiel 2

### (±)-3-(1-Methyl-1-methoxyethoxy)tetradecanal (rac-VIII)

Der nach Beispiel 1 erhaltene rohe Ester wird in 30 ml Toluol gelöst, unter Argon auf -80 °C abgekühlt und im Verlaufe von 1 h bei einer Temperatur von -80 bis -90 °C mit 30 ml einer 1-molaren Lösung von Diisobutylaluminiumhydrid in Hexan versetzt. Anschließend rührt man noch 30 min bei dieser Temperatur, versetzt mit 10 ml Methanol und läßt das Gemisch auf Raumtemperatur kommen. Nach Zugabe von 25 ml gesättigter Natriumchlorid-Lösung filtriert man den gebildeten Niederschlag ab, wäscht ihn fünfmal mit je 10 ml Essigsäureethylester, trennt die Phasen und extrahiert die wäßrige Phase noch dreimal mit je 15 ml Essigsäureethylester. Die vereinigten Extrakte werden mit 10 ml Wasser gewaschen, über Natriumsulfat getrocknet und unter verminderten Druck vom Lösungsmittel befreit. Das als Rückstand verbleibende (±)-3-(1-Methyl-1-methoxyethoxy)tetradecanal ist für die Weiterverarbeitung genügend rein [¹³C-NMR (500 MHz) einer analysenreinen Probe: δ = 13.85, 22.05, 24.49, 24.87, 24.96, 28.68, 28.94, 28.97, 29.00, 29.04, 31.28, 35.56, 48.50, 48.93, 66.25, 100.48, 202.80. - C₁₈H₃₆O₃ (300.5): berechnet C 71.95, H 12.08, gefunden C 72.05, H 12.28)].

### Beispiel 3

### (3RS,4RS)-3-Hexyl-4-[(2RS)-2-hydroxytridecyl]oxetan-2-on (rac-IX) und (3RS,4RS)-3-Hexyl-4-[(2SR)-2-hydroxytridecyl]oxetan-2-on (rac-X)

Der nach Beispiel 2 erhaltene rohe Aldehyd wird in 6 ml Tetrahydrofuran gelöst, auf -50 °C abgekühlt und im Verlaufe von 60 min zu einer auf -90 °C abgekühlten Lösung des Lithiumenolats des 1-Octanoylbenzotriazols in Tetrahydrofuran getropft. Das Reaktionsgemisch wird 30 min bei -90 °C gerührt und dann innerhalb von 4 h auf Raumtemperatur erwärmt. Danach werden 25 ml 2 N Salzsäure unter Kühlung mit Eis hinzugefügt und zur Abspaltung der Schutzgruppe 20 min gerührt. Nach Zugabe von 20 ml Diethylether werden die wäßrige und die organische Phase getrennt. Die wäßrige Phase wird dreimal mit je 20 ml Diethylether extrahiert. Die organische Phase und die Extrakte werden vereinigt, über Natriumsulfat getrocknet und nach Abfiltrieren des Trockenmittels unter vermindertem Druck vom Lösungsmittel befreit.

Durch Flashchromatographie des Rückstandes an Kieselgel mit Hexan/Essigsäureethylester (4:1) werden (3*RS*,4*RS*)-3-Hexyl-4-[(2*RS*)-2-hydroxytridecyl]oxetan-2-on (*rac-***IX**) und (3*RS*,4*RS*)-3-Hexyl-4-[(2*SR*)-2-hydroxytridecyl]oxetan-2-on (*rac-***X**) gewonnen. Eine weitere Reinigung der Fraktionen erfolgt durch erneute Chromatographie oder durch Umkristallisation aus Pentan.

Zur Herstellung des in diesem Beispiel verwendeten Lithiumenolats des 1-Octanoylbenzotriazols wird eine auf -20 °C abgekühlte Lösung von 5.40 g (22 mmol) 1-Octanoylbenzotriazol in 6 ml Tetrahydrofuran bei -90 °C innerhalb von 30 min zu einer Lösung von Lithiumhexamethyldisilazid getropft, die durch Zugabe von 15.55 ml einer 1.4-normalen Lösung von Butyllithium in Hexan zu einer Lösung von 5.05 ml Hexamethyldisilazan in 30 ml Tetrahydrofuran gewonnen wurde.

Nach dieser Vorschrift wurden erhalten:
(3*RS*,4*RS*)-3-Hexyl-4-[(2*RS*)-2-Hydroxytridecyl]oxetan-2-on (*rac-***IX**): Farblose, kristalline Substanz; Fp. 49-50 °C; ¹H-NMR (500 MHz, C₆D₆): δ = 0.83-0.93 (m, 6 H), 1.05-1.40 (m, 31 H), 1.45-1.53 (m, 1 H), 1.55-1.62 (m, 1 H), 2.84-2.88 (m, 1 H), 3.30-3.37 (m, 1 H), 4.06-4.12 (m, 1 H); ¹³C-NMR (125 MHz, CDCl₃): δ = 14.07, 14.17, 22.59, 22.74, 25.52, 26.87, 27.88, 29.02, 29.41, 29.59, 29.63 (2 Signale), 29.69, 29.71, 31.57, 31.98, 37.74, 41.24, 56.87, 69.43, 76.39, 171.67.
(3*RS*,4*RS*)-3-Hexyl-4-[(2*SR*)-2-hydroxytridecyl]oxetan-2-on (*rac*-**X**): Farblose kristalline Substanz; Fp. 44-46 °C; ¹H-NMR (500 MHz, CDCl₃): δ = 0.86-0.95 (m, 6 H), 1.22-1.97 (m, 33 H), 3.24-3.30 (m, 1 H), 3.76-3.85 (m, 1 H), 4.48-4.54 (m, 1 H); ¹³C-NMR (125 MHz, CDCl₃): δ = 14.07, 14,16, 22.57, 22.73, 25.45, 26.82, 27.76, 29.03, 29.40, 29.55, 29.62 (2 Signale), 29.68, 29.70, 31.57, 31.98, 38.18, 41.90, 56.67, 68.62, 75.73, 171.96.

### Beispiel 4

### (3S,4S)-3-Hexyl-4-[(2S)-2-hydroxytridecyl]oxetan-2-on (IX) und (3R,4R)-3-Hexyl)-4-[(2S)-2-hydroxytridecyl]oxetan-2-on (X)

Ausgehend von 5.16 g (20 mmol) (*S*)-3-Hydroxytetradecansäure-methylester (**VII**) werden in einer Reaktionsfolge in Analogie zu den Beispielen 1, 2 und 3 (3*S*,4*S*)-3-Hexyl-4-[(2*S*)-2-hydroxytridecyl)oxetan-2-on (**IX**) und (3*R*,4*R*)-3-Hexyl-4-[(2*S*)-2-hydroxytridecyl)oxetan-2-on (**X**) erhalten.
(3*S*,4*S*)-3-Hexyl-4-[(2*S*)-2-hydroxytridecyl]oxetan-2-on (**IX**): farblose, kristalline Verbindung; Fp. 63-64 °C; ¹H-NMR: identisch mit dem von *rac-***IX** (Beispiel 3).
(3*R*,4*R*)-3-Hexyl-4-[(2*S*)-2-hydroxytridecyl]oxetan-2-on (**X**): farblose, kristalline Substanz; Fp. 59-60 °C; ¹H-NMR: identisch mit dem von *rac-***X** (Beispiel 3).

### Beispiel 5

### (3RS,4SR)-3-Hexyl-4-[(1SR)-1-hydroxypentadecyl]oxetan-2-on (rac-XI)

Ausgehend von 5.73 g (20 mmol) (*RS*)-2-Hydroxyhexadecansäure-methylester wird in einer Reaktionsfolge in Analogie zu den Beispielen 1, 2 und 3 (3*RS*,4*SR*)-3-Hexyl-4-[(1*SR*)-1-hydroxypentadecyl]oxetan-2-on (*rac*-**XI**) erhalten. Farblose, kristalline Verbindung; Fp. 40-41 °C; ¹H-NMR (500 MHz, CDCl₃): δ = 0.86-0.91 (m, 6 H), 1.23-1.57 (m, 35 H), 1.65-1.76 (m, 1 H), 1.79-1.87 (m, 1 H), 3.60-3.65 (m, 1 H), 3.97-4,02 (m, 1 H), 4.14-4.16 (m, 1 H); ¹³C-NMR (125 MHz, CDCl₃): δ = 14.00, 14,09, 22,51, 22.68, 25.45, 26.97, 27.68, 28.91, 29.34, 29.45 (2 Signale), 29.54, 29.61, 29.64-29.67 (4 Signale), 31.50, 31.91, 32.11, 50.55, 69.80, 79.29, 171.29.

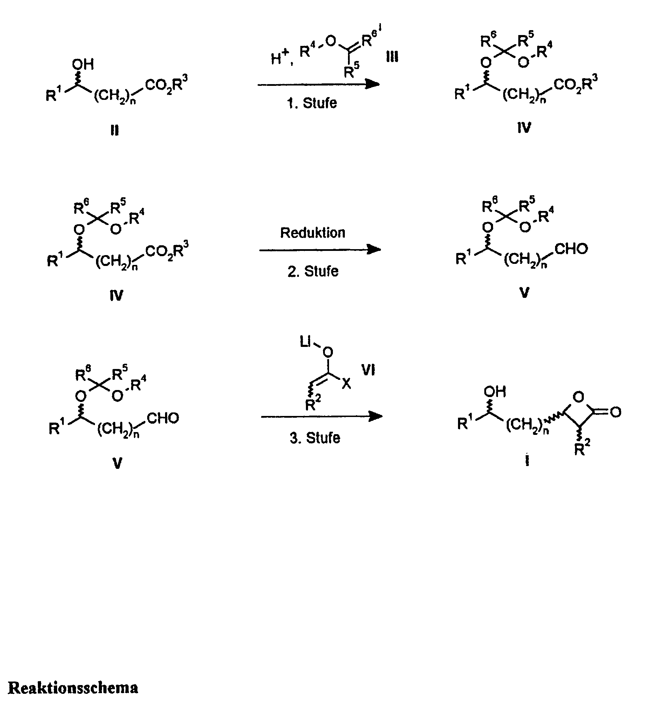

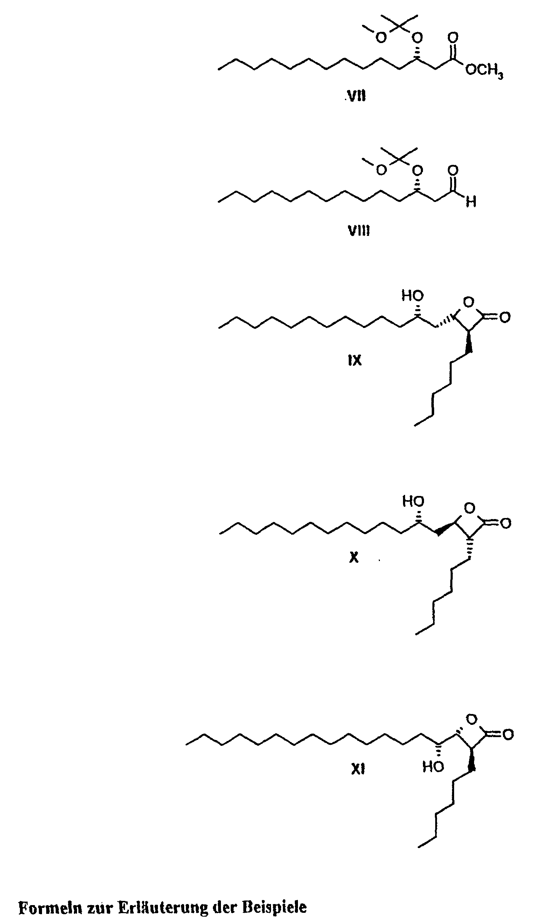

## Patentansprüche

1. Verfahren zur Herstellung von Verbindungen der allgemeinen Formel **I** 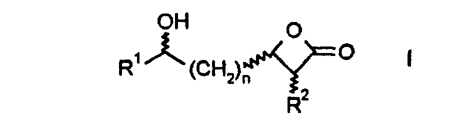 in der R¹ einen Alkyl-, Alkenyl-, Alkadienyl- oder Alkinylrest mit bis zu 20 Kohlenstoffatomen bedeutet, in dem die Kohlenstoffkette durch 1 bis 3 Halogenatome, eine Alkylgruppe, eine Arylgruppe oder eine Arylalkylgruppe substituiert oder durch ein Sauerstoffatom, ein Schwefelatom oder eine 1,4-Arylengruppe unterbrochen sein kann, in der R² eine Arylamino-, Alkoxy-, Aryloxy-, Alkylsulfanyl-, Arylsulfanyl- oder Halogengruppe oder einen Alkyl-, Alkenyl-, Alkadienyl- oder Alkinylrest mit bis zu 20 Kohlenstoffatomen bedeutet, in dem die Kohlenstoffkette durch 1 bis 3 Halogenatome, eine Alkylgruppe, eine Arylgruppe oder eine Arylalkylgruppe substituiert oder durch ein Sauerstoffatom, ein Schwefelatom oder eine 1,4-Arylengruppe unterbrochen sein kann, und in der n die Bedeutung null oder 1 hat,
**dadurch gekennzeichnet, daß** ein Ester der allgemeinen Formel **II** 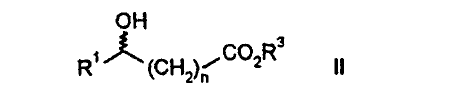 in der R¹ die oben genannte Bedeutung hat
und R³ Alkyl, vorzugsweise Methyl oder Ethyl, bedeutet,
mit Enolethern der allgemeinen Formel **III** 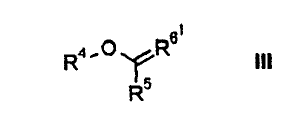 in der R⁴ Alkyl, vorzugsweise Methyl oder Ethyl,
R⁵ Wasserstoff oder Alkyl, vorzugsweise Methyl oder Ethyl,
R^{6'} Alkyliden, vorzugsweise Methylen oder Ethyliden
oder R⁵ und R^{6'} zusammen Alkyliden, vorzugsweise CH(CH₂)_{m'} mit m'= 3 bis 14 bedeuten,
in einer säurekatalysierten Reaktion zu einer Verbindung der allgemeinen Formel **IV** 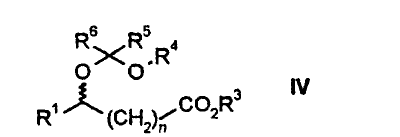 umgesetzt wird,
in der R¹, R³, R⁴, R⁵ und n die oben genannte Bedeutung haben,
R⁶ Alkyl, vorzugsweise Methyl oder Ethyl,
oder R⁵ und R⁶ zusammen (CH₂)ₘ mit m = 4 bis 15 bedeuten,
dann die Verbindung der allgemeinen Formel **IV** durch Reduktion in einem organischen Lösungsmittel wie Toluol oder Dichlormethan, vorzugsweise mit Diisobutylaluminiumhydrid, in einen Aldehyd der Formel **V** 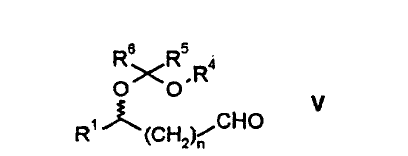 überführt wird,
in der R¹, R⁴, R⁵, R⁶ und n die oben genannte Bedeutung haben,
anschließend mit einem Metallenolat der allgemeinen Formel **VI** 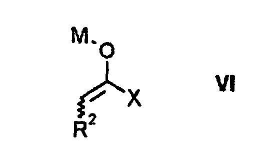 in der R² die oben angegebene Bedeutung hat,
X Fluor oder eine gegebenenfalls substituierte Aryloxy-, Arylsulfanyl- oder stickstoffhaltige Heteroarylgruppe, vorzugsweise eine 1-Benzotriazolyl- oder Phenoxygruppe ist,
und M für das Äquivalent eines ein-, zwei-, drei- oder vierwertiges Metalls wie Li, MgBr, ZnCl oder Ti(OPr^{*i*})₃ steht,
zu Verbindungen der allgemeinen Formel **I** 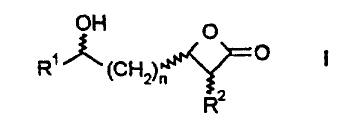 umgesetzt wird,
in der R¹, R² und n die oben angegebene Bedeutung haben,
die wahlweise durch chromatographische Methoden oder durch Umkristallisation in diastereomerenreine racemische oder enantiomerenreine Verbindungen getrennt werden .

2. Verfahren zur Herstellung von Verbindungen der allgemeinen Formel **I** nach Anspruch 1, **dadurch gekennzeichnet, daß** diese Verbindungen als Gemisch von Diastereomeren oder in diastereomerenreiner, enantiomerenreiner oder racemischer Form erhalten werden.

3. Verfahren nach Anspruch **1**, **dadurch gekennzeichnet, daß** eine Verbindung der allgemeinen Formel **II** mit 2-Methoxypropen in Gegenwart von Pyridinium-*p*-toluolsulfonat mit oder ohne organisches Lösungsmittel bei einer Temperatur zwischen -20 und 35 °C, vorzugsweise bei 20 bis 25 °C umgesetzt wird.

4. Verfahren nach Anspruch 1, **dadurch gekennzeichnet, daß** die Reduktion eines Esters der allgemeinen Formel **IV** zu einem Aldehyd der allgemeinen Formel **V** mit Diisobutylaluminiumhydrid bei einer Temperatur von -20 bis -90 °C, vorzugsweise bei -70 °C durchgeführt wird.

5. Verfahren nach Anspruch 1, **dadurch gekennzeichnet, daß** die Umsetzung eines Aldehyds der allgemeinen Formel **V** mit einem Metallenolat der allgemeinen Formel **VI** zu einem Oxetan-2-on der allgemeinen Formel **I** bei einer Temperatur von -20 bis -100 °C, vorzugsweise bei - 90 °C erfolgt.

6. Verbindungen der allgemeinen Formel **IV** 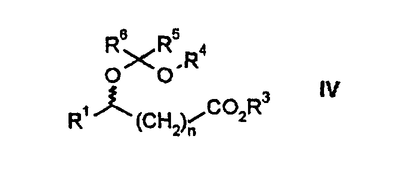 in der R¹ einen Alkyl-, Alkenyl-, Alkadienyl- oder Alkinylrest mit 4 bis 20 Kohlenstoffatomen bedeutet, in dem die Kohlenstoffkette durch 1 bis 3 Halogenatome, eine Alkylgruppe, eine Arylgruppe oder eine Arylalkylgruppe substituiert oder durch ein Sauerstoffatom, ein Schwefelatom oder eine 1,4-Arylengruppe unterbrochen sein kann,
in der R³ Alkyl, vorzugsweise Methyl oder Ethyl, bedeutet,
in der R⁴, R⁵ und R⁶ Alkyl, vorzugsweise Methyl oder Ethyl, bedeuten
oder in der R⁵ und R⁶ zusammen (CH₂)ₘ mit m = 4 bis 15 bedeuten
und in der n 0 oder 1 bedeutet.

7. Verbindungen nach Anspruch 6 als Gemische von Diastereomeren oder in diastereomerenreiner, enantiomerenreiner oder racemischer Form.

8. Verbindungen der allgemeinen Formel **V** 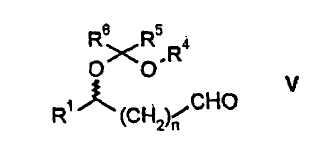 in der R¹ einen Alkyl-, Alkenyl-, Alkadienyl- oder Alkinylrest mit 4 bis 20 Kohlenstoffatomen bedeutet, in dem die Kohlenstoffkette durch 1 bis 3 Halogenatome, eine Alkylgruppe, eine Arylgruppe oder eine Arylalkylgruppe substituiert oder durch ein Sauerstoffatom, ein Schwefelatom oder eine 1,4-Arylengruppe unterbrochen sein kann,
in der R⁴, R⁵ und R⁶ Alkyl, vorzugsweise Methyl oder Ethyl, bedeuten
oder in der R⁵ und R⁶ zusammen (CH₂)ₘ mit m = 4 bis 15 bedeuten
und in der n 0 oder 1 bedeutet.

9. Verbindungen nach Anspruch 8 als Gemische von Diastereomeren oder in diastereomerenreiner, enantiomerenreiner oder racemischer Form.

## Claims

1. A process for the preparation of compounds of the general formula **I**: 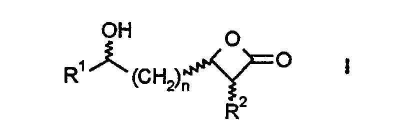 wherein R¹ is alkyl, alkenyl, alkadienyl or alkynyl having up to 20 carbon atoms, in which the carbon chain may be substituted by 1 to 3 halogen atoms, an alkyl, aryl or aryl alkyl group or may be interrupted by an oxygen atom, a sulfur atom or a 1,4-arylene group;
wherein R² is arylamino, alkoxy, aryloxy, alkylsulfanyl, arylsulfanyl, or halogen or is alkyl, alkenyl, alkadienyl, or alkynyl having up to 20 carbon atoms, in which the carbon chain may be substituted by 1 to 3 halogen atoms, an alkyl, aryl or aryl alkyl group or may be interrupted by an oxygen atom, a sulfur atom or a 1,4-arylene group; and
wherein n is 0 or 1,
which comprises the reaction of an ester of the general general formula **II:** 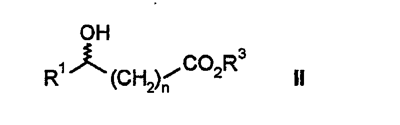 wherein R¹ has the same meaning as above; and
wherein R³ is alkyl, preferably methyl or ethyl,
with an enol ether of the general formula **III:** 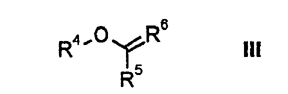 wherein R⁴ is alkyl, preferably methyl or ethyl;
wherein R⁵ is H or alkyl, preferably methyl or ethyl;
wherein R^{6'} is an alkylidene group, preferably a methylene or ethylidene group; or
wherein R⁵ and R⁶ together represent an alkylidene group, preferably =CH(CH₂)_{m'}-, wherein m' is an integer between 3 and 14,
in an acid catalyzed reaction to a compound of the general formula **IV**: 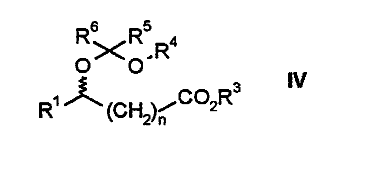 wherein R¹, R³, R⁴, R⁵, and n have the same meaning as above;
wherein R⁶ is alkyl, preferably methyl or ethyl; or
wherein R⁵ and R⁶ together represent -(CH₂)ₘ-, wherein m is an integer between 4 and 15,
followed by reduction of this compound of the general formula **IV** in an organic solvent such as toluene or dichloromethane, preferably with diisobutylaluminiumhydride, to an aldehyde of the general formula **V**: 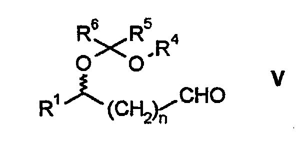 wherein R¹, R⁴, R⁵, R⁶, and n have the same meaning as above,
and condensation of this aldehyde with a metal enolate of the general formula **VI:** 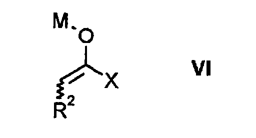 wherein R² has the same meaning as above;
wherein X is fluorine, substituted or unsubstituted aryloxy, arylsulfanyl, or N containing heteroaryl, preferably phenoxy and 1-benzotriazolyl; and
wherein M stands for an equivalent of a one-, two-, three- or four-valent metal, such as Li, MgBr, ZnCl or Ti(OPr^{*i*})₃,
to compounds of the general formula **I**: 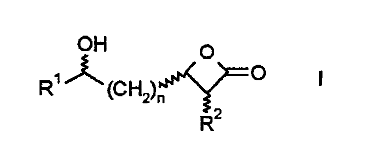 wherein R¹, R², and n have the same meaning as above,
which are separated by chromatography or recrystallization into diastereomerically pure racemic or enantiomerically pure compounds.

2. The process of claim **1** for the preparation of compounds of the general formula **I** wherein these compounds are obtained as a mixture of diastereoisomers or in diastereoisomerically pure, enantiomerically pure or racemic form.

3. The process of claim **1** wherein a compound of the general formula **II** is reacted with 2-methoxypropen in the presence of pyridinium *p*-toluenesulfonate with or without an organic solvent at a temperature between -20 and +35°C, preferably at a temperature of 20-25 °C.

4. The process of claim **1** wherein the reduction of an ester of the general formula **IV** to an aldehyde of the general formula **V** is performed with diisobutylaluminium hydride at a temperature between -20 and -90 °C, preferably at - 70 °C.

5. The process of claim **1** wherein the reaction of the aldehyde of the general formula **V** with a metal enolate of the general formula **VI** to an oxetanone of the general formula **I** is performed at a temperature between -20 and -100 °C, preferably at - 90 °C.

6. The compounds of the general formula **IV**: 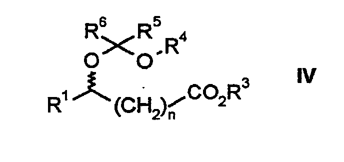 wherein R¹ is alkyl, alkenyl, alkadienyl or alkynyl having 4 to 20 carbon atoms, in which the carbon chain may be substituted by 1 to 3 halogen atoms, an alkyl, aryl or aryl alkyl group or may be interrupted by an oxygen atom, a sulfur atom or a 1,4-arylene group;
wherein R³ is alkyl, preferably methyl or ethyl;
wherein R⁴, R⁵, and R⁶ is alkyl, preferably methyl or ethyl; or
wherein R⁵ and R⁶ together represent an alkyl chain of the general formula - (CH₂)ₘ-, wherein m is an integer between 4 and 15; and
wherein n is 0 or 1.

7. The compounds of claim **6** as mixtures of diastereoisomers or in diastereoisomerically pure, enantiomerically pure or racemic form.

8. The compounds of the general formula **V**: 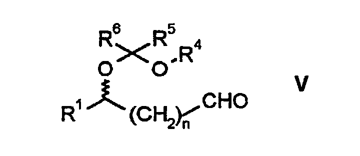 wherein R¹ is alkyl, alkenyl, alkadienyl or alkynyl having 4 to 20 carbon atoms, in which the carbon chain may be substituted by 1 to 3 halogen atoms, an alkyl, aryl or aryl alkyl group or may be interrupted by an oxygen atom, a sulfur atom or a 1,4-arylene group;
wherein R⁴, R⁵, and R⁶ are alkyl, preferably methyl or ethyl; or
wherein R⁵ and R⁶ together represent an alkyl chain of the general formula - (CH₂)ₘ-, wherein m is an integer between 4 and 15; and
wherein n is 0 or 1.

9. The compounds of claim **8** as mixtures of diastereoisomers or in form of diastereoisomerically pure compounds, enantiomerically pure compounds or racemates.

## Revendications

1. Procédé de préparation de composés de formule générale I 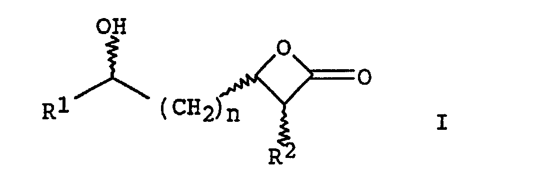 dans laquelle R¹ représente un radical alkyle, alcènyle, alcadiènyle ou alkinyle ayant jusqu'à 20 atomes de carbone, dans lequel la chaîne carbonée peut être substituée par 1 à 3 atomes d'halogène, un groupe alkyle, un groupe aryle ou un groupe arylalkyle ou être interrompue par un atome d'oxygène, un atome de soufre ou un groupe 1,4-arylène, dans laquelle R² représente un groupe arylamino, alcoxy, aryloxy, alkylsulfanyle, arylsulfanyle ou un groupe halogène ou un radical alkyle, alcènyle, alcadiènyle ou alkinyle ayant jusqu'à 20 atomes de carbone, dans lequel la chaîne carbonée peut être substituée par 1 à 3 atomes d'halogène, un groupe alkyle, un groupe aryle ou un groupe arylalkyle ou être interrompue par un atome d'oxygène, un atome de soufre ou un groupe 1,4-arylène, dans laquelle n signifie 0 ou 1,
**caractérisé en ce qu'**un ester de formule générale II 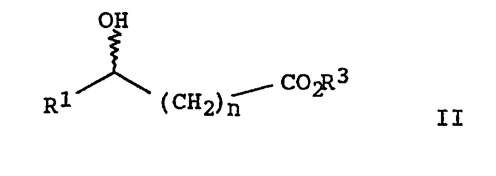 dans laquelle R¹ a la signification susmentionnée
et R³ représente un alkyle, de préférence un méthyle ou un éthyle,
est transformé avec des énoléthers de formule générale III : 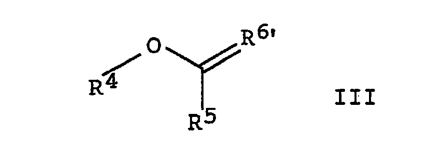 dans laquelle R⁴ représente un alkyle, de préférence un méthyle ou un éthyle,
R⁵ un hydrogène ou un alkyle, de préférence un méthyle ou un éthyle,
R⁶' un alkylidène, de préférence un méthylène ou un éthylidène,
ou R⁵ et R⁶' ensemble représentent un alkylidène, de préférence CH(CH₂)_{m'} avec m' = 3 à 14,
dans une réaction à catalyse acide en un composé de formule générale IV : 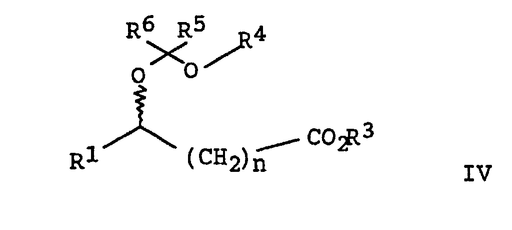 dans laquelle R¹, R³, R⁴ et R⁵ ont la signification susmentionnée,
R⁶ représente un alkyle, de préférence un méthyle ou un éthyle,
ou R⁵ et R⁶ ensemble représentent (CH₂)ₘ avec m = 4 à 15,
le composé de formule générale IV est ensuite transformé par réduction dans un solvant organique comme le toluène ou le dichlorométhane, de préférence avec de l'hydrure de diisobutylaluminium, en un aldéhyde de formule V : 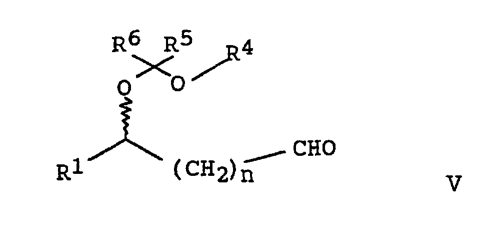 dans laquelle R¹, R⁴, R⁵, R⁶ et n ont la signification susmentionnée,
par la suite, converti avec un métallènolate de formule générale VI 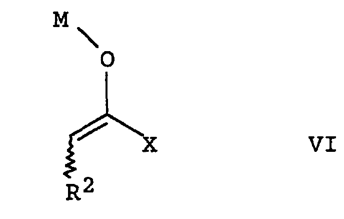 dans laquelle R² a la signification susmentionnée,
X représente le fluor ou un groupe aryloxy, alylsulfanyle ou hétéroaryle azoté éventuellement substitué, de préférence un groupe 1-benzotrizolyle ou phénoxy,
et M est l'équivalent d'un métal mono, di, tri ou quadrivalent comme Li, MgBr, ZnCl ou Ti(Oprⁱ)₃,
en des composés de formule générale I 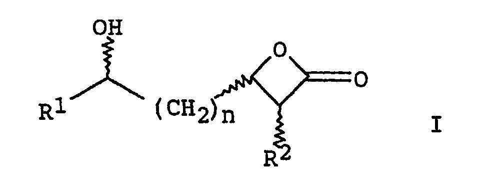 dans laquelle R¹, R² et n ont la signification susmentionnée,
qui sont séparés sélectivement par des méthodes chromatographiques ou par transcristallisation en composés racémiques purs de diastéréomères ou purs d'énantiomères.

2. Procédé de préparation de composés de formule générale I selon la revendication 1, **caractérisé en ce que** ces composés sont obtenus sous forme de mélange de diastéréomères ou sous la forme pure de diastéréomères, pure d'énantiomères ou racémique.

3. Procédé selon la revendication 1, **caractérisé en ce qu'**un composé de formule générale II est transformé avec du 2-méthoxypropène en présence de pyridinium-p-toluènesulfonate avec ou sans solvant organique à une température située entre -20°C et 35°C, de préférence entre 20 et 25°C.

4. Procédé selon la revendication 1, **caractérisé en ce que** la réduction d'un ester de formule générale IV en un aldéhyde de formule générale V est réalisée avec de l'hydrure de diisobutylaluminium à une témpérature de -20 à -90°C, de préférence à -70°C.

5. Procédé selon la revendication 1, **caractérisé en ce que** la transformation d'un aldéhyde de formule générale V avec un métallènolate de formule générale VI en un oxétan-2-one de formule générale I s'effectue à une température de -20 à -100°C, de préférence à -90°C.

6. Composés de formule générale IV 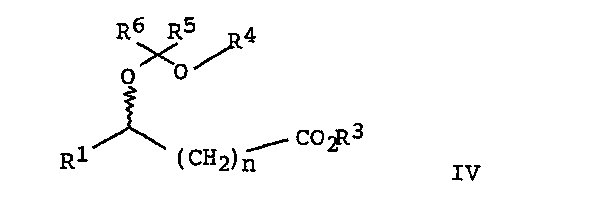 alcadiènyle ou alkinyle doté de 4 à 20 atomes de carbone, dans lequel la chaîne carbonée peut être substituée par 1 à 3 atomes d'halogène, un groupe alkyle, un groupe aryle ou un groupe arylalkyle ou être interrompue par un atome d'oxygène, un atome de soufre ou un groupe 1,4-arylène,
dans laquelle R³ représente un alkyle, de préférence un méthyle ou un éthyle,
dans laquelle R⁴, R⁵ et R⁶ représentent un alkyle, de préférence un méthyle ou un éthyle,
ou dans laquelle R⁵ et R⁶ ensemble représentent (CH₂)ₘ avec m = 4 à 15,
et dans laquelle n signifie 0 ou 1.

7. Composés selon la revendication 6 sous forme de mélanges de diastéréomères ou sous la forme pure de diastéréomères, pure d'énantiomères ou racémique.

8. Composés de formule générale V 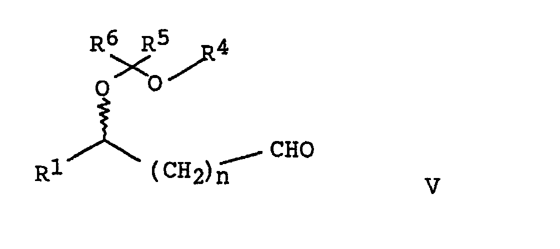 dans laquelle R¹ représente un radical alkyle, alcènyle, alcadiènyle ou alkinyle doté de 4 à 20 atomes de carbone, dans lequel la chaîne carbonée peut être substituée par 1 à 3 atomes d'halogène, un groupe alkyle, un groupe aryle ou un groupe arylalkyle ou être interrompue par un atome d'oxygène, un atome de soufre ou un groupe 1,4-arylène,
dans laquelle R⁴, R⁵ et R⁶ représentent un alkyle, de préférence un méthyle ou un éthyle,
ou dans laquelle R⁵ et R⁶ ensemble représentent (CH₂)ₘ avec m = 4 à 15,
et dans laquelle n signifie 0 ou 1.

9. Composés selon la revendication 8 sous forme de mélanges de diastéréomères ou sous la forme pure de diastéréomères, pure d'énantiomères ou racémique.
